(19) Europäisches Patentamt
European Patent Office
Office européen des brevets

(11) EP 1 059 280 A2

(12) # EUROPÄISCHE PATENTANMELDUNG

(43) Veröffentlichungstag:
**13.12.2000 Patentblatt 2000/50**

(21) Anmeldenummer: **00110999.0**

(22) Anmeldetag: **30.05.2000**

(51) Int. Cl.[7]: **C07C 45/49**, C07C 47/47, C07C 47/457, C07C 29/14, C07C 33/46, C07C 253/30

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **11.06.1999 DE 19926621**

(71) Anmelder: **BAYER AG**
**51368 Leverkusen (DE)**

(72) Erfinder:
- **Neugebauer, Torsten, Dr.**
  **Cambridge, MA 02139 (US)**
- **Marhold, Albrecht, Dr.**
  **51373 Leverkusen (DE)**

(54) **Verfahren zur Herstellung von Benzylalkoholen und deren Verwendung**

(57) Benzylalkohole, insbesondere solche, die Fluor- oder Fluoralkylsubstituenten am Benzylring tragen, lassen sich durch Formylierung entsprechender Arylbromide zu Benzaldehyden und deren Reduktion mit weiterem Formiat erhalten, wobei die gebildeten Benzaldehyde nicht isoliert werden müssen.



Printed by Xerox (UK) Business Services
2.16.7 (HRS)/3.6

**Beschreibung**

Die vorliegende Erfindung betrifft ein Verfahren zur Herstellung von Benzylalkoholen durch Formylierung von Arylbromiden zu Benzaldehyden und deren Reduktion mit einem Formiat.

Es ist bereits bekannt, Arylbromide in Gegenwart von Natriumformiat und eines Palladiumkatalysators bei Normaldruck mit Kohlenmonoxid zu formylieren (Bull. Chem. Soc. Jpn., 67, 2329-2332, 1994). Die entsprechenden Benzaldehyde können zum Teil in sehr guten Ausbeuten erhalten werden. Das Entstehen von Benzylalkoholen ist jedoch nicht erwähnt.

Obwohl verschiedene Synthesemethoden für Benzylalkohole bekannt sind, steht kein genereller Weg zur Verfügung, der vorteilhaft für die industrielle Produktion verschiedenster substituierter Benzylalkohole Einsatz finden könnte. In besonderem Maße gilt dies für die Darstellung von Benzylalkoholen, die am aromatischen Kern Fluor- oder fluorhaltige Substituenten tragen.

Beispielsweise lehrt US 4 118 561, 4-(Trifluormethoxy)benzylalkohol durch Reduktion von 4-(Trifluormethoxy)benzoesäure mit Lithiumalanat herzustellen. In J. Med. Chem. 18, 1216, 1975 ist die Reduktion der 4-(Trifluormethoxy)benzoesäure mit Diboran beschrieben. Die Durchführung von Reduktionen mit Lithiumalanat oder Diboran in technischem Maßstab würde erheblichen sicherheitstechnischen Aufwand erfordern.

Benzylalkohole finden unter anderem Verwendung bei der Herstellung von Lacken und Aromen und können zur Darstellung von Phenethylaminen verwendet werden. Phenethylamine mit Fluor- oder fluorhaltigen Substituenten, zum Beispiel 4-(Tri-fluormethoxy)phenethylamin, sind interessante Zwischenprodukte, beispielsweise für Agrochemikalien (EP 00 665 225 oder JP 08 291 149).

Es besteht Bedarf an einem einfachen und sicheren Verfahren zur Darstellung von Benzylalkoholen mit möglichst variablem Substitutionsmuster, insbesondere solcher, die Fluor- oder fluorhaltige Substituenten tragen.

Es wurde nun ein Verfahren zur Herstellung von Benzylalkoholen der Formel (I)

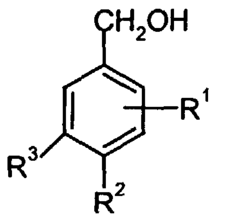

(I)

in der

$R^1$, $R^2$ und $R^3$ jeweils unabhängig voneinander H, F, Cl, $NO_2$, geradkettiges oder verzweigtes $C_1$-$C_{12}$-Alkyl, Phenyl, OH, $O(CH_2)_nCH_3$ mit n = 0-12, $(CF_2)_nCF_3$ mit n = 0-2 oder $O(CX_2)_nCX_3$ mit n = 0-12, wobei X für F und/oder Cl steht, bedeuten,

gefunden, das dadurch gekennzeichnet ist, dass man Arylbromide der allgemeinen Formel (II)

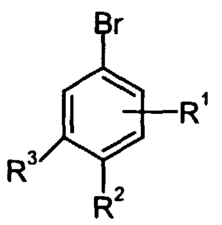

(II)

wobei

$R^1$, $R^2$, $R^3$ die bei Formel (I) angegebene Bedeutung haben,

in Gegenwart eines Palladiumkatalysators mit Kohlenmonoxid und einem Formiat zu Benzaldehyden der Formel (III)

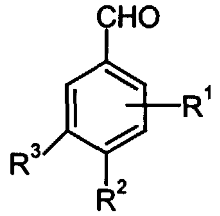

(III)

umsetzt, wobei

$R^1$, $R^2$ und $R^3$ die bei Formel (I) angegebene Bedeutung haben,
und dass man die so erhaltenen Benzaldehyde der Formel (III) zu den entsprechenden Benzylalkoholen reduziert.

Die gebildeten Benzaldehyde der Formel (III) können gegebenenfalls isoliert, gereinigt und in einem zweiten Schritt reduziert werden.

In den Formeln (I), (II), (III) und (IV) steht $R^1$ vorzugsweise in meta-Position zur $CH_2OH$-, Br-, CHO- bzw. $CH_2$-$CH_2$-$NH_2$-Gruppe.

Die Reduktion zu Benzylalkoholen der Formel (I) kann mit Formiat in Gegenwart eines Palladiumkatalysators erfolgen. Alternativ lässt sich gebildeter Benzaldehyd auch mit Wasserstoff in einem Autoklaven bei Temperaturen zwischen 60°C und 120°C und Drücken zwischen 30 bar und 70 bar in Gegenwart eines Palladiumkatalysators zum Benzylalkohol reduzieren. Die Reduktion mit Wasserstoff stellt insbesondere dann eine interessante Alternative dar, wenn Benzylalkohole der Formel (I), in denen mindestens einer der Reste $R^1$, $R^2$ oder $R^3$ F, $CF_3$, $CF_2CF_3$, $CF_2CF_2CF_3$ oder $O(CX_2)_nCX_3$, mit n = 0-12 und X = F und/oder Cl, ist, hergestellt werden sollen, beispielsweise 4-Trifluormethoxybenzylalkohol.

Bevorzugt wird das erfindungsgemäße Verfahren jedoch als Eintopfreaktion durchgeführt, wobei die im ersten Schritt gebildeten Benzaldehyde der Formel (III) ohne zwischengeschaltete Aufarbeitung direkt mit Formiat an einem Palladiumkatalysator zu Benzylalkoholen der Formel (I) umgesetzt werden. Bei dieser Vorgehensweise entfallen Reinigungsschritte auf der Stufe der Benzaldehyde, was den Syntheseaufwand im Vergleich zu zweistufigen Verfahren deutlich verringert und dieses Vorgehen damit aus verfahrenstechnischen und ökonomischen Gründen sehr interresant macht.

Das verwendete Formiat kann beispielsweise in Form des Ammonium-, Lithium-, Kalium-, Natrium-, Caesium-, Calcium-, oder Bariumsalzes eingesetzt oder in situ, beispielsweise aus Triethylamin und Ameisensäure, erzeugt werden. Die Verwendung von Kalium-, Natrium- oder Ammoniumformiat ist bevorzugt.

Zur Durchführung des erfindungsgemäßen Verfahrens wird ein Palladiumkatalysator verwendet. Vorteilhaft wird Palladium in der Oxidationsstufe 0 oder II eingesetzt.

Als Katalysatoren, in denen Palladium in der Oxidationsstufe 0 vorliegt, können beispielsweise Palladiumkomplexe mit gegebenenfalls substituierten Triphenylphosphin-Liganden verwendet werden. Als Beispiel sei hier Tetrakistriphenylphosphinpalladium(0) genannt.

Katalysatoren, in denen Palladium in der Oxidationsstufe II vorliegt, können in Form von Palladium(II)-Salzen in Verbindung mit geeigneten freien Liganden oder in Form von Palladium(II)-Komplexen eingesetzt werden.

Geeignete Palladium(II)-Komplexe sind zum Beispiel Verbindungen der allgemeinen Formeln

$$L_2PdX_2 \text{ oder } L^1PdX_2,$$

wobei

- L für Phosphin mit gegebenenfalls substituierten, gegebenenfalls verzweigten C1-C10-Alkyl- und/oder gegebenenfalls substituierten $C_6$-$C_{10}$-Arylresten,
- $L^1$ für Chelatliganden der Formel $R^4{}_2P(CH_2)nPR^4{}_2$, mit $R^4$ = gegebenenfalls substituiertes, gegebenenfalls verzweigtes C1-C10-Alkyl oder gegebenenfalls substituiertes $C_6$-$C_{10}$-Aryl und n = 1-4,
  und
- X für Cl, Br, I, Acetat, Dibenzylidenaceton und/oder Nitrat steht.

Als Liganden L seien beispielhaft $PPh_3$, $P(2\text{-}MeC_6H_4)_3$, $P(4\text{-}MeC_6H_4)_3$, $P(4\text{-}MeOC_6H_4)_3$, $P(4\text{-}ClC_6H_4)_3$, $P(cyclohexyl)_3$, $PMe_2Ph$, $PPh_2Me$, $PPh_2(CH(CH_3)_2)$, $PEt_3$, $PMe_3$, $P(CH(CH_3)_2)_3$ und $P(C(CH_3)_3)_3$ genannt. Dabei kön-

nen die beiden Liganden L jeweils unabhängig voneinander gewählt werden. Vorteilhaft sind die beiden Liganden L jedoch gleich.

Als Chelatliganden $L^1$ seien beispielhaft $(C_6H_5)_2PCH_2P(C_6H_5)_2$, $(C_6H_5)_2P(CH_2)_2P(C_6H_5)_2$, $(C_6H_5)_2P(CH_2)_3P(C_6H_5)_2$ und 1,1'-Bis(diphenylphosphino)ferrocen genannt.

Als Palladium(II)-Salze in Verbindung mit freien Liganden L oder $L^1$, wobei L und $L^1$ die oben angegebene Bedeutung haben, kommen beispielsweise Palladium(II)-halogenide, insbesondere Palladium(II)-chlorid, Palladium(II)-acetylacetonat, $Pd(CN)_2$, $Pd(CH_3CN)Cl_2$, $Pd(C_6H_5CN)Cl_2$, Pd(II)-1,1,1,5,5,5-hexafluoroacetylacetonat und Palladium(II)-acetat in Frage.

Das erfindungsgemäße Verfahren wird vorteilhaft bei Temperaturen von 80°C bis 140°C, bevorzugt bei Temperaturen von 105°C bis 115°C durchgeführt.

Der Reaktionsdruck ist nicht kritisch. Will man bei hohen Temperaturen mit niedrig siedenden Lösungsmitteln arbeiten, kann ein Autoklav verwendet werden. Vorteilhaft wird jedoch in einem Druckbereich von über 1 bar, vorzugsweise von 1 bar bis 10 bar gearbeitet. In einer bevorzugten Ausführungsform wird Kohlenmonoxid so in die Reaktionsmischung eingeleitet, dass der Reaktionsdruck annähernd Atmosphärendruck (0,8 bar bis 1,2 bar) beträgt. Durch eine solche Reaktionsführung sind die Ansprüche an die Druckstabilität des verwendeten Reaktors gering. Für die Durchführung des erfindungsgemäßen Verfahren sind demnach eine Vielzahl von Reaktoren geeignet.

Sind die eingesetzten Arylbromide unter den Reaktionsbedingungen flüssig, kann das erfindungsgemäße Verfahren ohne Zugabe eines Lösungsmittels durchgeführt werden. Vorteilhaft wird jedoch in einem polaren, aprotischen, organischen Lösungsmittel, beispielsweise Dimethylformamid, N-Methylpyrrolidon, Dimethylacetamid, Pyridin, 1,3-Dimethylimidazolidin-2-on, Dimethylsulfoxid, Toluol, Xylol, Acetonitril, Propionitril, Benzonitril oder Chlorbenzol oder Gemischen davon gearbeitet. Bevorzugte Lösungsmittel sind Dimethylformamid, N-Methylpyrrolidon und Dimethylacetamid.

Zur Durchführung des erfindungsgemäßen Verfahrens kann beispielsweise so vorgegangen werden, dass man das umzusetzende Arylbromid zusammen mit einem Palladiumkatalysator und einem Formiat in einem Reaktionsgefäß vorlegt, diese Mischung mit einem inerten Lösungsmittel versetzt und unter Einleiten von CO bei Atmosphärendruck und erhöhter Temperatur zunächst zum Benzaldehyd umsetzt. Anschließend beendet man das Einleiten von CO und lässt den Benzaldehyd zum Benzylalkohol weiterreagieren.

Überraschenderweise lassen sich nach dem erfindungsgemäßen Verfahren Arylbromide durch Reaktion mit Kohlenmonoxid und Formiat, einem sehr schwachen Reduktionsmittel, in einer Eintopfreaktion zu Benzylalkoholen umsetzen, während das Gemisch Kohlenmonoxid und Formiat bislang lediglich dazu verwendet wurde, aus Arylbromiden Benzaldehyde darzustellen.

Besonders vorteilhaft eignet sich das erfindungsgemäße Verfahren zur Herstellung von Benzylalkoholen der Formel (I), in denen mindestens einer der Reste $R^1$ $R^2$ oder $R^3$ F, $CF_3$, $CF_2CF_3$, $CF_2CF_2CF_3$ oder $O(CX_2)_nCX_3$, mit n = 0-12 und X = F und/oder Cl, ist, insbesondere zur Herstellung von 4-Trifluormethoxybenzylalkohol.

Die erfindungsgemäß dargestellten Benzylalkohole der Formel (I) können zur Herstellung von Phenethylaminen der Formel (IV)

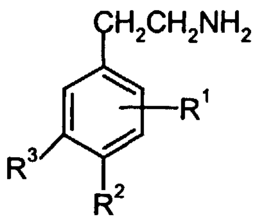

(IV)

wobei

$R^1$, $R^2$ und $R^3$ die bei Formel (I) angegebene Bedeutung haben,

verwendet werden. Geeignete Reagenzien und Reaktionsbedingungen sind dem Fachmann bekannt. Die erfindungsgemäß dargestellten Benzylalkohole können, wie beispielsweise in US 4118561 beschrieben, zunächst mit einem üblichen Halogenierungsreagenz, etwa Thionylchlorid, zu Benzylhalogeniden umgesetzt werden. Diese lassen sich, etwa durch Umsetzung mit Alkalicyanid (vgl. Ullmann's Encyclopedia of Industrial Chemistry, Bd. A17, 5. Ausgabe, 1991, S. 372) zu Benzylcyaniden und diese schließlich durch Hydrierung, beispielsweise mit Lithiumalanat und Aluminiumtrichlorid oder Wasserstoff in Gegenwart eines Cobaltkatalysators (Beilsteins Handbuch der Organischen Chemie, 4. Auflage 1984, Band E IV 12, S. 2453), in Phenethylamine der Formel (IV) überführen.

**Beispiele**

**Beispiel 1**

4-Trifluormethoxybenzaldehyd

In einem Kolben mit Rückflusskühler und Gaseinleitungsrohr wurden 3,62 g 4-Trifluormethoxybrombenzol, 0,21 g Bis(triphenylphosphin)palladiumdichlorid und 1,53 g Natriumformiat vorgelegt, mit 15 ml DMF versetzt, gerührt und unter Einleiten von CO auf 110°C erhitzt. Nach vollständigem Umsatz (GC-Kontrolle) ließ man die Reaktionsmischung auf 21°C abkühlen und trennte den Katalysator durch Filtration über Kieselgel ab.
Es wurde ein Rohprodukt gewonnen, das 91 % an 4-Trifluormethoxybenzaldehyd und 7 % an 4-Trifluormethoxybenzylalkohol enthielt (Prozentangaben sind bezogen auf die Flächenanteile im GC).

**Beispiel 2**

4-Trifluormethylbenzaldehyd

In einem Kolben mit Rückflusskühler und Gaseinleitungsrobr wurden 3,38 g 4-Trifluormethylbrombenzol, 0,21 g Bis(triphenylphosphin)palladiumdichlorid und 1,53 g Natriumformiat vorgelegt, mit 15 ml DMF versetzt, gerührt und unter Einleiten von CO auf 110°C erhitzt. Nach vollständigem Umsatz (GC-Kontrolle) ließ man die Reaktionsmischung auf 21°C abkühlen und trennte den Katalysator durch Filtration über Kieselgel ab.
Es wurde ein Rohprodukt gewonnen, das 91 % an 4-Trifluormethylbenzaldehyd und 8 % an 4-Trifluormethylbenzylalkohol enthielt (Prozentangaben sind bezogen auf die Flächenanteile im GC).

**Beispiel 3**

3,4-Difluorbenzaldehyd

In einem Kolben mit Rückflusskühler und Gaseinleitungsrohr wurden 2,90 g 3,4-Difluorbrombenzol, 0,21 g Bis(triphenylphosphin)palladiumdichlorid und 1,53 g Natriumformiat vorgelegt, mit 15 ml DMF versetzt, gerührt und unter Einleiten von CO auf 110°C erhitzt. Nach vollständigem Umsatz (GC-Kontrolle) ließ man die Reaktionsmischung auf 21°C abkühlen und trennte den Katalysator durch Filtration über Kieselgel ab.
Es wurde ein Rohprodukt gewonnen, das 95 % an 3,4-Difluorbenzaldehyd und 4 % an 3,4-Difluorbenzylalkohol enthielt (Prozentangaben sind bezogen auf die Flächenanteile im GC).

**Beispiel 4**

2-Fluor-4-methylbenzaldehyd

In einem Kolben mit Rückflusskühler und Gaseinleitungsrohr wurden 2,84 g 2-Fluor-4-methylbrombenzol, 0,21 g Bis(triphenylphosphin)palladiumdichlorid und 1,53 g Natriumformiat vorgelegt, mit 15 ml DMF versetzt, gerührt und unter Einleiten von CO auf 110°C erhitzt. Nach vollständigem Umsatz (GC-Kontrolle) ließ man die Reaktionsmischung auf 21°C abkühlen und trennte den Katalysator durch Filtration über Kieselgel ab.
Es wurde ein Rohprodukt gewonnen, dessen Anteil an 2-Fluor-4-methylbenzaldehyd >95 % und an 2-Fluor-4-methylbenzylalkohol <1 % war (Prozentangaben sind bezogen auf die Flächenanteile im GC).

**Beispiel 5**

4-Trifluormethoxybenzylalkohol

In einem Kolben mit Rückflusskühler wurden 8,55 g 4-Trifluormethoxybenzaldehyd, 0,63 g Bis(triphenylphosphin)palladiumdichlorid und 6,12 g Natriumformiat vorgelegt, mit 45 ml DMF versetzt, gerührt und auf 110°C erhitzt. Der Reaktionsverlauf wurde mittels GC kontrolliert. Nach vollständigem Umsatz (GC-Kontrolle) ließ man die Reaktionsmischung auf 21°C abkühlen und trennte den Katalysator durch Filtration über Kieselgel ab. Es wurde mit Methyltert.butylether nachgespült und mit 3 x 100 ml Wasser gewaschen, uni das DMF zu entfernen. Man erhielt 6,0 g (69 % der Theorie) 4-Trifluormethoxybenzylalkohol.

**Beispiel 6**

3,5-Bis-(trifluormethyl)-benzaldehyd

In einem Kolben mit Rückflusskühler und Gaseinleitungsrohr wurden 4,40 g 3,5-Bis-(trifluormethyl)-brombenzol, 0,21 g Bis(triphenylphosphin)palladiumdichlorid und 1,53 g Natriumformiat vorgelegt, mit 15 ml DMF versetzt, gerührt und unter Einleiten von CO auf 110°C erhitzt. Nach vollständigem Umsatz (GC-Kontrolle) ließ man die Reaktionsmischung auf 21°C abkühlen und trennte den Katalysator durch Filtration über Kieselgel ab.

Es wurde ein Rohprodukt gewonnen, dessen Anteil an 3,5-Bis-(trifluormethyl)-benzaldehyd 43 % und an 3,5-Bis-(trifluormethyl)-benzylalkohol 57 % war (Prozentangaben sind bezogen auf die Flächenanteile im GC).

**Beispiel 7**

4-Fluor-3-methyl-benzaldehyd

In einem Kolben mit Rückflusskühler und Gaseinleitungsrohr wurden 2,85 g 5-Brom-2-fluortoluol, 0,21 g Bis(triphenylphosphin)palladiumdichlorid und 1,53 g Natriumformiat vorgelegt, mit 15 ml DMF versetzt, gerührt und unter Einleiten von CO auf 110°C erhitzt. Nach vollständigem Umsatz (GC-Kontrolle) ließ man die Reaktionsmischung auf 21°C abkühlen und trennte den Katalysator durch Filtration über Kieselgel ab.

Es wurde ein Rohprodukt gewonnen, dessen Anteil an 4-Fluor-3-methyl-benzaldehyd >98 % war (Prozentangabe ist bezogen auf die Flächenanteile im GC). Es ließ sich kein Edukt mehr nachweisen.

**Patentansprüche**

1. Verfahren zur Herstellung von Benzylalkoholen der Formel (I)

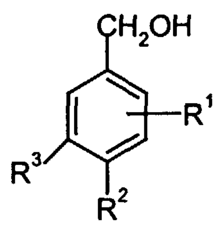

(I)

in der

$R^1$, $R^2$ und $R^3$ jeweils unabhängig voneinander H, F, Cl, $NO_2$, geradkettiges oder verzweigtes $C_1$-$C_{12}$-Alkyl, Phenyl, OH, $O(CH_2)_nCH_3$ mit n = 0-12, $(CF_2)_nCF_3$ mit n = 0-2 oder $O(CX_2)_nCX_3$ mit n = 0-12, wobei X für F und/oder Cl steht, bedeuten,
dadurch gekennzeichnet, dass man Arylbromide der allgemeinen Formel (II)

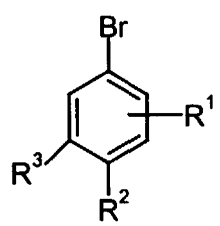

(II)

wobei

$R^1$, $R^2$, $R^3$ die bei Formel (I) angegebene Bedeutung haben,
in Gegenwart eines Palladiumkatalysators mit Kohlenmonoxid und einem Formiat zu Benzaldehyden der Formel (III)

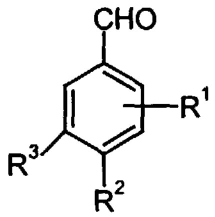

(III)

umsetzt, wobei

$R^1$, $R^2$ und $R^3$ die bei Formel (I) angegebene Bedeutung haben und dass man

die Benzaldehyde der Formel (III) zu den entsprechenden Benzylalkoholen reduziert.

**2.** Verfahren nach Anspruch 1, dadurch gekennzeichnet, dass die Reduktion der Benzaldehyde der Formel (III) mit Wasserstoff in Gegenwart eines Palladiumkatalysators erfolgt.

**3.** Verfahren nach Anspruch 1, dadurch gekennzeichnet, dass die Reduktion der Benzaldehyde der Formel (III) mit weiterem Formiat in Gegenwart eines Palladiumkatalysators erfolgt.

**4.** Verfahren nach mindestens einem der Ansprüche 1 und 3, dadurch gekennzeichnet, dass die Benzaldehyde der Formel (III) ohne zwischengeschaltete Aufarbeitung direkt mit weiterem Formiat in Gegenwart eines Palladiumkatalysators zu Benzylalkoholen der Formel (I) umgesetzt werden.

**5.** Verfahren nach mindestens einem der Ansprüche 1 bis 4, dadurch gekennzeichnet, dass Formiat in Form seines Ammonium-, Lithium-, Kalium-, Natrium-, Caesium-, Calcium-, oder Bariumsalzes eingesetzt oder in situ erzeugt wird.

**6.** Verfahren nach mindestens einem der Ansprüche 1 bis 5, dadurch gekennzeichnet, dass als Katalysator Tetrakistriphenylphosphinpalladium(0) verwendet wird.

**7.** Verfahren nach mindestens einem der Ansprüche 1 bis 6, dadurch gekennzeichnet, dass als Katalysator ein Palladiumkomplex der allgemeinen Formel $L_2PdX_2$ verwendet wird, wobei

L für Phosphin mit substituierten oder unsubstituierten C1-C10-Alkyl- und/oder substituierten oder unsubstituierten $C_6$-$C_{10}$-Arylresten, und X für Cl, Br, I, Acetat, Dibenzylidenaceton und/oder Nitrat steht.

**8.** Verfahren nach mindestens einem der Ansprüche 1 bis 7, dadurch gekennzeichnet, dass als Katalysator ein Palladiumkomplex der allgemeinen Formel $L^1PdX_2$ verwendet wird, wobei

$L^1$ für Chelatliganden der Formel $R^4{}_2P(CH_2)_nPR^4{}_2$, mit $R^4$= substituiertes oder unsubstituiertes C1-C10-Alkyl oder substituiertes oder unsubstituiertes $C_6$-$C_{10}$-Aryl und n = 1-4,
und X für Cl, Br, I, Acetat, Dibenzylidenaceton und/oder Nitrat steht.

**9.** Verfahren nach mindestens einem der Ansprüche 1 bis 8, dadurch gekennzeichnet, dass als Katalysator Palladium(II)-Salze in Verbindung mit freien Liganden L oder $L^1$ eingesetzt werden, wobei L und $L^1$ die oben angegebene Bedeutung haben.

**10.** Verfahren nach mindestens einem der Ansprüche 1 bis 9, dadurch gekennzeichnet, dass in einem Temperaturbereich von 80°C bis 140°C gearbeitet wird.

**11.** Verfahren nach mindestens einem der Ansprüche 1 bis 10, dadurch gekennzeichnet, dass in einem Druckbereich von 1 bar bis 10 bar gearbeitet wird.

**12.** Verfahren nach mindestens einem der Ansprüche 1 bis 11, dadurch gekennzeichnet, dass es in einem polaren,

aprotischen, organischen Lösungsmittel durchgeführt wird.

**13.** Verfahren nach mindestens einem der Ansprüche 1 bis 12, dadurch gekennzeichnet, dass es in Dimethylformamid, N-Methylpyrrolidon oder Dimethylacetamid durchgeführt wird.

**14.** Verfahren nach mindestens einem der Ansprüche 1 bis 13, dadurch gekennzeichnet, dass 4-Trifluormethoxybrombenzol umgesetzt wird.

**15.** Verwendung der entsprechend den Ansprüchen 1 bis 13 dargestellten Benzylalkohole der Formel (I) zur Herstellung von Phenethylaminen der Formel (IV)

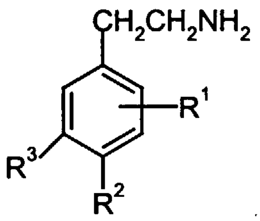

(IV)

wobei

$R^1$, $R^2$ und $R^3$ die bei Formel (I) angegebene Bedeutung haben,

dadurch gekennzeichnet, dass die erfindungsgemäß dargestellten Benzylalkohole zunächst mit einem üblichen Halogenierungsreagenz zu Benzylhalogeniden, dann durch Umsetzung mit Cyanid zu Benzylcyaniden umgesetzt und schließlich zu Phenethylaminen der Formel (IV) reduziert werden.